# FASCICULE DE BREVET EUROPEEN

(11) **EP 3 236 841 B1**
(45) Date de publication et mention de la délivrance du brevet: **26.06.2019**
(21) Numéro de dépôt: 15821142.5
(22) Date de dépôt: 16.12.2015
(51) Int. Cl.: A61B 5/026, A61B 5/1455, A61B 5/00, G01N 21/64, A61B 5/02, G01N 21/47

(54) **DISPOSITIF D'IMAGERIE ET PROCÉDÉ D'IMAGERIE**
BILDGEBUNGSVORRICHTUNG UND -VERFAHREN
IMAGING DEVICE AND METHOD

(30) Priorité: 24.12.2014 FR 1463335
(43) Date de publication de la demande: 01.11.2017
(73) Titulaire: SORBONNE UNIVERSITE, 75006 Paris (FR); Centre National de la Recherche Scientifique (CNRS), 75016 Paris (FR); INSERM (Institut National de la Santé et de la Recherche Médicale), 75013 Paris (FR); Université Paris-Sud, 91405 Orsay Cedex (FR); Assistance Publique-Hôpitaux de Paris (APHP), 75100 Paris (FR)
(72) Inventeur: CHENEGROS, Guillaume, 78190 Trappes (FR); BENOSMAN, Ryad, 75016 Paris (FR); LIBERT, Nicolas, 75005 Paris (FR); DURENTEAU, Jacques, 75016 Paris (FR); HARROIS, Anatole, 94120 Fontenay Sous Bois (FR); PICAUD, Serge, 77210 Avon (FR); SAHEL, José-Alain, 75012 Paris (FR)
(74) Mandataire: Cabinet Plasseraud
(86) Numéro de dépôt international: PCT/FR2015/053537
(87) Numéro de publication internationale: WO 2016/102819

(56) Documents cités:
- CN-A- 103 356 174
- CN-A- 103 445 764
- CN-B- 101 900 875
- US-A1- 2006 184 037

## Description

La présente invention se rapporte à des dispositifs d'imagerie, et plus particulièrement à des dispositifs d'imagerie pour l'étude de la microcirculation sanguine.

Différentes techniques d'imagerie vidéo-microscopique ont été développées pour la surveillance de la microcirculation et plus généralement pour l'étude de la circulation des vaisseaux sanguins de diamètre de l'ordre du µm. Ces techniques permettent de visualiser par exemple le déplacement des cellules sanguines, et en particulier celui des globules rouges dans les vaisseaux sanguins, en particulier les capillaires sanguins.

La technique OPS, pour Orthogonal Polarization Spectral, utilise un faisceau de lumière incidente, polarisée linéairement dans un plan, qui est projeté sur le sujet au travers d'un « beam splitter ». L'analyse est effectuée sur la lumière réfléchie dont la polarisation est modifiée selon le degré de profondeur de pénétration dans les tissus de la lumière incidente avant d'être réfléchie. Une large part de la lumière réfléchie est issue de la réflexion par les couches supérieures des tissus observés, et conserve sa polarisation initiale, tandis que la lumière incidente qui pénètre plus profondément dans les tissus observés, subit de ce fait plus de « scattering » et perd sa polarisation initiale. Le dispositif d'analyse comprend une caméra devant l'objectif de laquelle est disposé un polarisateur orthogonal qui vient bloquer la portion de lumière réfléchie qui a conservé sa polarisation, afin de générer une image à partir de la lumière réfléchie non filtrée par le polariseur.

La technique SDF, pour Sidestream Dark Field, utilise une illumination en champ sombre (en anglais, « dark field »), fournie par une lentille prismatique circulaire disposée autour de l'objectif d'une caméra, afin de générer un halo de lumière autour et au-delà du point focal de l'objectif. La caméra est typiquement située au-dessus d'une zone centrale du halo lumineux de manière à capter la lumière rétrodiffusée. Ce type d'illumination, qui permet une profondeur d'observation plus importante et une imagerie en trois dimensions des tissus visualisés, semble bien adaptée pour l'analyse de la microcirculation sanguine.

Un exemple de dispositif d'imagerie de la microcirculation fonctionnelle utilisant le principe de la microscopie en champ sombre est décrit dans la demande internationale publiée sous le numéro WO 2005/032361 A2. La demande CN 103 356 174 divulgue un dispositif d'imagerie medicale pour l'imagerie microscopique a deux photons et l'enregistrement synchrone electro-physiologique.

Les dispositifs d'imagerie de la microcirculation fonctionnelle existants ne permettent cependant pas l'observation de phénomènes rapides tels que la circulation des globules rouges. Ils présentent en outre des limitations pour l'observation et l'analyse en temps-réel de la microcirculation.

Il existe ainsi un besoin pour des dispositifs d'imagerie ne présentant pas les inconvénients des dispositifs classiques exposés ci-dessus. En particulier, un premier besoin est de fournir des dispositifs d'imagerie qui permettent une observation de phénomènes rapides liés à la microcirculation sanguine, tels que le déplacement des globules rouges. Un autre besoin est de fournir des dispositifs d'imagerie qui permettent un traitement en temps-réel d'informations générées afin d'analyser la microcirculation sanguine.

Selon un premier aspect, il est proposé un dispositif d'imagerie comprenant au moins une source de lumière agencée pour générer un signal optique, un support optique couplé à la source de lumière et agencé pour projeter un signal d'excitation lumineux d'intensité lumineuse sensiblement constante issu de la source de lumière vers un corps à observer lors de l'utilisation du dispositif, et une première caméra asynchrone couplée au support optique et configurée pour générer un signal comprenant, pour chaque pixel d'une première matrice de pixels, une séquence de signal représentant des évènements asynchrones correspondant à des variations de la lumière rétrodiffusée par le corps à observer pour le pixel.

L'utilisation d'une caméra asynchrone pour l'imagerie de phénomènes rapides, notamment biologiques, présente de nombreux avantages. Ceux-ci résultent notamment du fait que l'acquisition vidéo n'est pas pilotée par une horloge, mais par les évènements survenant pour chaque pixel du capteur dans la scène en regard de laquelle est placé le capteur. Les caméras classiques sont à l'inverse par des signaux de contrôle et de synchronisation générés par le capteur à partir d'une fréquence d'horloge, signaux qui n'ont aucun lien avec la source de l'information visuelle. Ce mode de fonctionnement radicalement différent des caméras asynchrones permet d'atteindre des valeurs de résolution temporelles beaucoup plus élevées que celles des caméras classiques, ce qui permet la mise en oeuvre de systèmes d'imagerie ayant des résolutions temporelles et spatiales élevées avec une sensibilité suffisante pour ouvrir un nouveau champ des possibles dans l'observation et l'analyse de phénomènes biologiques rapides tels que la microcirculation.

En outre, les procédés d'acquisition ou de synthèse d'une séquence d'images par trames présentent l'inconvénient de produire des données avec une forte redondance, due au fait que chaque trame représente un grand nombre de pixels d'une image si ce n'est une image entière, et que l'ensemble de ces pixels pour lesquels l'information ne change pas d'une image à l'autre génère des redondances dans les données représentant la séquence d'images. Cette redondance ne peut être qu'en partie supprimée par un encodage de compression d'un signal vidéo classique. A l'inverse, les signaux asynchrones générés par les caméras asynchrones permettent d'obtenir une représentation très compacte de données relatives à une séquence d'images, du fait que ces données, représentant des évènements pour un pixel (et non pas pour tous les pixels d'une matrice ou un grand nombre d'entre eux), ne sont pas redondantes d'une image à l'autre.

Dans un mode de réalisation, le dispositif d'imagerie peut comprendre une deuxième caméra asynchrone couplée au support optique et configurée pour générer un signal comprenant, pour chaque pixel d'une deuxième matrice de pixels, une séquence de signal représentant des évènements asynchrones correspondant à des variations de la lumière rétrodiffusée par le corps à observer pour le pixel, et dans lequel un premier filtre optique est agencé pour filtrer la lumière rétrodiffusée captée par la première caméra asynchrone.

L'utilisation d'une deuxième caméra asynchrone permet certains types d'analyses, comme l'analyse spectrale, qui utilise les propriétés de la lumière rétrodiffusée acquise pour une même observation pour différentes longueurs d'onde.

En variante, le premier filtre optique peut être utilisé pour filtrer la lumière rétrodiffusée captée par la première caméra asynchrone, et un deuxième filtre optique peut en outre être agencé pour filtrer la lumière rétrodiffusée captée par la deuxième caméra asynchrone. Cela permet de sélectionner la lumière en fonction de sa longueur d'onde afin que chaque caméra asynchrone reçoive sensiblement simultanément l'information provenant du corps étudié.

Dans un mode de réalisation, le dispositif d'imagerie peut comprendre une troisième caméra asynchrone couplée au support optique et configurée pour générer un signal comprenant, pour chaque pixel d'une troisième matrice de pixels, une séquence de signal représentant des évènements asynchrones correspondant à des variations de la lumière rétrodiffusée par le corps à observer pour le pixel, et dans lequel un troisième filtre optique est agencé pour filtrer la lumière rétrodiffusée captée par la troisième caméra asynchrone.

Dans un mode de réalisation, la source de lumière peut être une diode électroluminescente, par exemple stimulée par une tension continue, ou bien une source laser.

Dans un mode de réalisation, le dispositif peut comprendre une pluralité de sources de lumière agencées sur le support optique pour générer des signaux d'excitation lumineux d'intensités lumineuses respectives sensiblement constantes.. L'intensité lumineuse peut être différente d'une source lumineuse à l'autre, et le mode de réalisation dans lequel les sources de lumière sont de même intensité lumineuse sensiblement constante est un exemple non limitatif de réalisation du dispositif proposé.

De plus, les sources de lumière pourront être agencées sur le support optique de manière à former un anneau lumineux.

Dans un mode de réalisation, la ou les caméras asynchrones peuvent être agencées sur le support situées au-dessus d'une zone non illuminée par la/les sources de lumière.

Selon un autre aspect, un système d'imagerie de la microcirculation comprenant un dispositif selon les différents modes de réalisation décrits dans les présentes est proposé.

Selon un autre aspect, il est proposé un procédé d'imagerie comprenant : générer un signal optique d'intensité lumineuse sensiblement constante ; projeter un signal d'excitation lumineux issu du signal optique vers un corps à observer ; et générer un signal comprenant, pour chaque pixel d'une première matrice de pixels, une séquence de signal représentant des évènements asynchrones correspondant à des variations de la lumière rétrodiffusée par le corps à observer pour le pixel.

D'autres particularités et avantages de la présente invention apparaîtront dans la description ci-après d'exemples de réalisation non limitatifs, en référence aux dessins annexés, dans lesquels :
La figure 1 est un schéma synoptique d'un dispositif d'imagerie selon un mode de réalisation du dispositif proposé;
La figure 2 est un schéma synoptique d'une caméra asynchrone d'un dispositif d'imagerie selon un mode de réalisation du dispositif proposé ;
La figure 3a est un diagramme montrant un exemple de profil d'intensité lumineuse au niveau d'un pixel d'un capteur asynchrone ;
La figure 3b montre un exemple de signal délivré par le capteur asynchrone en réponse au profil d'intensité de la figure 3a ;
La figure 3c illustre la reconstruction du profil d'intensité à partir du signal de la figure 3b ;
Les figures 4a-4b sont des diagrammes analogues à ceux des figures 3a et 3b illustrant un mode d'acquisition lumineuse utilisable dans un autre exemple de réalisation du procédé ;
La figure 5 est un schéma synoptique d'un dispositif d'imagerie selon un mode de réalisation du dispositif proposé ;
La figure 6 est un schéma synoptique d'une portion de dispositif d'imagerie selon un mode de réalisation du dispositif proposé ;
La figure 7a est un schéma synoptique d'un dispositif d'imagerie selon un mode de réalisation du dispositif proposé ;
La figure 7b est un schéma synoptique de portions du dispositif d'imagerie de la figure 7a ;
La figure 8 est un schéma synoptique d'un dispositif d'imagerie multi-caméras selon un mode de réalisation du dispositif proposé ;
Les figures 9a et 9b sont des schémas synoptiques de portions de dispositifs d'imagerie multi-caméras selon différents modes de réalisation du dispositif proposé ;
La figure 10 est un diagramme illustrant le procédé d'imagerie proposé selon un mode de réalisation.

Dans la description détaillée ci-après de modes de réalisation de l'invention, de nombreux détails spécifiques sont présentés pour apporter une compréhension plus complète. Néanmoins, l'homme du métier peut se rendre compte que des modes de réalisation peuvent être mis en pratique sans ces détails spécifiques. Dans d'autres cas, des caractéristiques bien connues ne sont pas décrites en détail pour éviter de compliquer inutilement la description.

Par « corps », on entend dans les présentes un tissu humain ou animal, dans le cadre par exemple de l'utilisation du dispositif proposé pour l'imagerie de la microcirculation, ou tout objet que l'on souhaite observer en utilisant le dispositif d'imagerie proposé ou le procédé d'imagerie proposé.

L'invention sera décrite ci-après dans le cadre non limitatif d'une information asynchrone représentant, pour un pixel d'une matrice de pixels, des évènements correspondant à des variations de lumière pour le pixel. Les dispositifs et systèmes proposés ne sont toutefois pas limités à ce mode de réalisation particulier, les évènements concernant le pixel pouvant selon le mode de réalisation correspondre à des variations de lumière pour le pixel, à la détection d'une forme d'intérêt ou à la détection d'une primitive, et plus généralement à tout type d'information asynchrone pour le pixel.

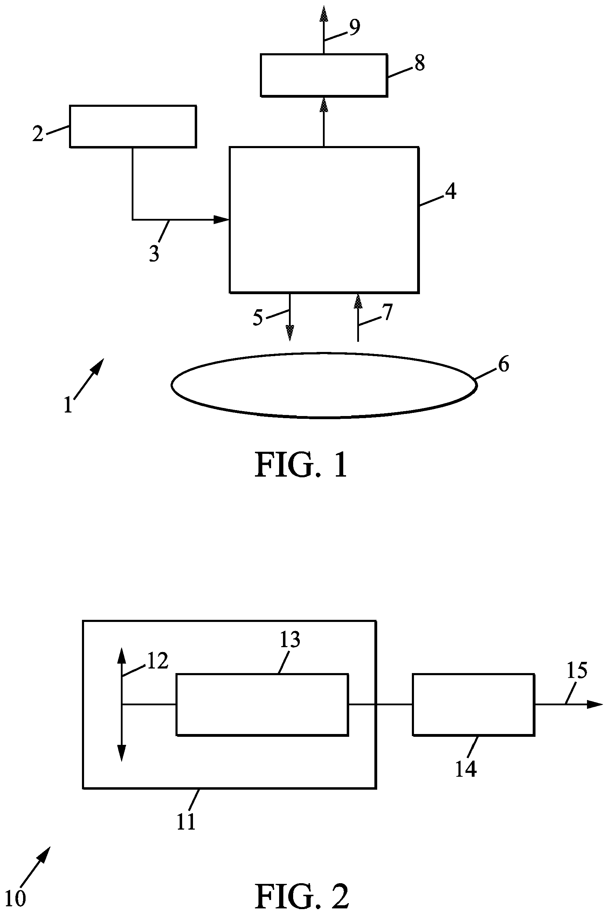

La figure 1 montre un diagramme d'un système d'imagerie selon un mode de réalisation. Le système d'imagerie (1) illustré comprend une source lumineuse (2) agencée pour émettre un signal optique (3). La source lumineuse (2) est couplée à un support optique (4) agencé pour projeter un signal optique d'excitation d'intensité lumineuse sensiblement constante (5) issu du signal (3) émis par la source en direction du corps (6) à analyser lors de l'utilisation du système (1).

Le support optique (4) est en outre agencé pour recevoir la lumière rétrodiffusée (7) par le corps (6) à analyser. Dans un mode de réalisation, la lumière rétrodiffusée peut subir différents traitements en fonction du type de traitements effectués post imagerie (traitements OPF, traitements SDF, etc.). Le support optique (4) est en outre couplé optiquement avec un capteur de vision asynchrone (8), de manière à ce que le capteur de vision asynchrone (8) puisse réagir à des évènements portés par le signal de lumière rétrodiffusée (7). Le capteur de vision asynchrone (8) est muni d'une interface (9) de sortie d'un signal asynchrone, sur lequel différents traitements peuvent être effectués pour l'application envisagée.

La source lumineuse (2) peut par exemple être de type diode électroluminescente alimentée en continu par une source électrique, une source laser, ou bien une source lumineuse incohérente.

Dans un mode de réalisation, un système de focalisation (non représentée sur la figure) est positionné dans le support optique (4) entre la source lumineuse (2) et le corps (6) à analyser afin de focaliser le signal optique d'excitation (5) projeté sur ce dernier. La distance focale du système de focalisation, pouvant par exemple comprendre une lentille, sera en pratique choisie en fonction de la résolution d'analyse souhaitée et de la source lumineuse utilisée.

Le signal optique d'excitation (5) est en partie absorbé par le corps (6) à analyser, qui rétrodiffuse de la lumière sous forme d'un signal de lumière rétrodiffusée (7). Le support optique (4) est agencé pour recevoir ce signal de lumière rétrodiffusée (7), et le transporter après d'éventuels traitements optiques vers le capteur de vision asynchrone (8).

La figure 2 illustre schématiquement une caméra asynchrone selon un mode de réalisation du système d'imagerie.

La caméra asynchrone (10) est apte à générer un signal portant de l'information asynchrone représentant, pour chaque pixel d'une matrice de pixels, des évènements correspondant respectivement à des variations de lumière captées par la caméra. Selon l'application envisagée, la caméra asynchrone (10) pourra être couplée de manière opérationnelle à un sous-système de visualisation et/ou à un sous-système de traitement et d'analyse, chaque sous-système comprenant une interface d'entrée configurée pour recevoir un signal portant de l'information asynchrone.

Le signal portant de l'information asynchrone transitant sur l'interface de couplage entre les sous-systèmes peut avoir différentes formes, ou formats, correspondants à différents modes de réalisation du système. L'interface de sortie de la caméra asynchrone (10) peut par ailleurs être prévue conforme à différents formats standards, tel que, par exemple, le format USB. Le système proposé n'est pas limité à un format particulier d'information asynchrone, de vecteur de cette information (par exemple, un signal asynchrone portant de l'information représentative d'un flux d'événements), ou un format spécifique d'interface de sortie de la caméra asynchrone (10).

Dans un mode de réalisation, le signal asynchrone généré en sortie de la caméra asynchrone (10) porte de l'information représentative d'évènements temporels correspondant à des variations de lumière captées par la caméra (10).

En référence à la figure 2, la caméra asynchrone (10) comprenant un dispositif d'acquisition de la lumière (11) comprenant un capteur de vision asynchrone basé sur évènements (13) placé en regard d'une scène à observer et recevant le flux lumineux de la scène à travers une optique d'acquisition (12). L'optique d'acquisition (12) pourra comprendre une lentille, dont les caractéristiques (notamment la focale et le diamètre) sont choisies adaptées aux caractéristiques du capteur (13). Le capteur (13) peut comporter un groupement d'éléments photosensibles organisés en une matrice de pixels, de sorte que chaque pixel de la matrice corresponde à un élément photosensible du capteur. Pour chaque pixel de la matrice, le dispositif (11) engendre une séquence de signal asynchrone basé sur évènement à partir des variations de lumière perçues par le pixel dans la scène apparaissant dans le champ de vision du dispositif (11). Chaque pixel correspondant à un élément photosensible, produit ainsi des évènements temporels correspondant respectivement à des variations de lumière.

Le capteur (13) ne produit donc pas des trames vidéo constituées par la matrice de pixels correspondant aux éléments photosensibles du capteur à une fréquence d'échantillonnage prédéterminée. Il réagit pour chaque pixel de la matrice à des évènements correspondant à des variations de lumière pour le pixel. A l'inverse, il ne produit pas d'information pour un pixel si aucun évènement ne se produit pour ce pixel. Il n'effectue en particulier pas de capture systématique d'intensité lumineuse des pixels de la matrice. Ainsi, les évènements auxquels il réagit sont asynchrones, et ne dépendent pas d'une fréquence d'acquisition de trames vidéo. La caméra (10) est ainsi asynchrone notamment en ce qu'elle ne nécessite pas de référence temporelle définissant un rythme d'acquisition des informations de niveau lumineux perçu par les pixels de la matrice. Chaque pixel envoie ses informations de manière asynchrone par rapport aux autres pixels de la matrice. Cela permet de diminuer fortement, si ce n'est supprimer, les redondances créées par l'acquisition de trames vidéo à un rythme déterminé ne tenant pas compte de l'absence de changement de l'information portée par un pixel d'une trame à l'autre.

A l'inverse, une caméra classique sera pilotée par un ensemble de signaux de synchronisation temporelle et de contrôle indépendants de la source de l'information visuelle, comme par exemple un signal d'horloge d'acquisition des pixels (opérant typiquement à un rythme de quelques MHz), dont le rythme définira une fréquence à laquelle l'acquisition d'un ensemble de pixels de la matrice sera effectuée. La résolution temporelle, correspondant à l'intervalle de temps minimum au cours duquel la caméra peut détecter un changement, sera donc beaucoup plus faible pour une caméra classique (par exemple de type CCD ou CMOS) (de l'ordre de la ms) que celle d'une caméra asynchrone (de l'ordre de la µs).

Une unité de traitement (14) traite l'information issue du capteur (13) et représentative des évènements produits de manière asynchrone par les différents pixels, pour générer un signal asynchrone (15) portant cette information.

Dans un mode de réalisation, le capteur (13) détecte indépendamment pour chaque pixel les changements relatifs, et initie de manière asynchrone et individuelle pour le pixel une mesure de valeur d'exposition ou de niveau de gris lorsque - et immédiatement après - un changement de luminosité d'une amplitude prédéterminée survient dans le champ de vision du pixel.

D'une manière générale, le capteur (13) génère des séquences respectives de signal asynchrone qui représentent des évènements relatifs aux pixels de la matrice. Dans un mode de réalisation particulier, chaque séquence de signal asynchrone indique, ou signale, des évènements relatifs à un pixel, indépendamment des autres séquences de signaux. Elle peut par exemple comprendre des données qui identifient des évènements avec leurs caractéristiques respectives.

Dans un mode de réalisation, l'unité de traitement (14) comprend un processeur couplé de manière opérationnelle à une mémoire. La mémoire peut contenir des instructions logicielles qui, lorsqu'elles sont exécutées par le processeur de l'unité de traitement des données amènent cette unité à traiter les signaux reçus du capteur et générer l'information asynchrone représentant pour chaque pixel des évènements correspondant à des variations de lumière concernant le pixel, et transmettre l'information asynchrone sur une interface de sortie. L'unité de traitement peut être un composant implémentant un processeur ou une unité de calcul pour la génération de l'information asynchrone selon les différents procédés décrits et le contrôle du capteur de vision asynchrone du dispositif (11) au sein de la caméra asynchrone (10).

En outre, l'unité de traitement, et en particulier son processeur et/ou ses moyens mémoire peuvent être, séparément ou conjointement, mis en oeuvre sous forme logicielle, comme décrit ci-dessus, sous forme matérielle, comme un circuit intégré spécifique application (ASIC), ou sous forme d'une combinaison d'éléments matériels et logiciels, comme par exemple un ou plusieurs programmes logiciel destinés à être chargés et exécutés respectivement sur un ou plusieurs composants de type FPGA (Field Programmable Gate Array). Ils peuvent être mis en oeuvre, séparément ou conjointement, sous forme d'un circuit électronique, ou bien au sein d'un ou de plusieurs composants électroniques (en anglais, chip ou chipset).

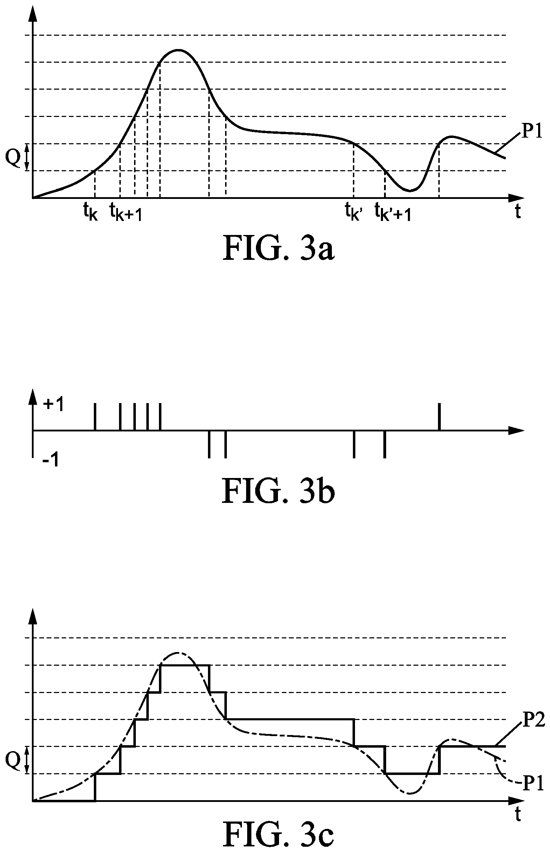

Un exemple de principe d'acquisition par capteur asynchrone est illustré par les figures 3a - 3c. Selon cet exemple, l'information consiste en une succession d'instants, notés *tₖ* (*k* = 0,1,2,...) auxquels un seuil d'activation Q est atteint. Le capteur (13) est donc muni d'un détecteur de variations qui, pour chaque pixel, mesure et enregistre l'intensité lumineuse du pixel lorsque cette intensité a varié au-delà d'un seuil Q.

La figure 3a montre un exemple de profil d'intensité lumineuse P1 vue par un pixel de la matrice du capteur de vision asynchrone. Chaque fois que cette intensité augmente d'une quantité égale au seuil d'activation Q en comparaison de ce qu'elle était à l'instant tₖ, un nouvel évènement est identifié et une raie positive (niveau +1 sur la figure 3b) est émise correspondant à l'instant de dépassement du seuil différentiel Q, noté tₖ₊₁. Symétriquement, chaque fois que l'intensité du pixel diminue de la quantité Q en comparaison de ce qu'elle était à l'instant t_{k'}, un nouvel évènement est identifié et une raie négative (niveau -1 sur la figure 3b) est émise correspondant à l'instant de dépassement du seuil différentiel Q, noté t_{k'+1}. La séquence de signal asynchrone pour le pixel consiste alors en une succession d'impulsions ou raies, positives ou négatives, positionnées dans le temps à des instants tₖ dépendant du profil lumineux pour le pixel. Ces raies peuvent être représentées mathématiquement par des pics de Dirac positifs ou négatifs et caractérisées chacune par un instant d'émission tₖ et un bit de signe. L'information correspondant à un évènement pour un pixel, peut comprendre une première information relative à un instant d'occurrence de l'événement, et une deuxième information relative à une caractéristique lumineuse pour le pixel à cet instant.

La forme de l'information asynchrone pour un pixel peut être différente d'une succession de pics de Dirac, les événements représentés pouvant avoir une largeur temporelle ou une amplitude ou une forme d'onde quelconque.

Le signal de sortie de la caméra asynchrone (10) correspond à ce qu'on appelle une représentation adresse-événement (AER, « address-event représentation ») : à chaque pixel correspond une séquence de signal basé sur évènement (« event based »).

La figure 3c montre le profil d'intensité P2 qu'on est capable de reconstruire comme une approximation du profil P1 par intégration dans le temps du signal asynchrone de la figure 3b.

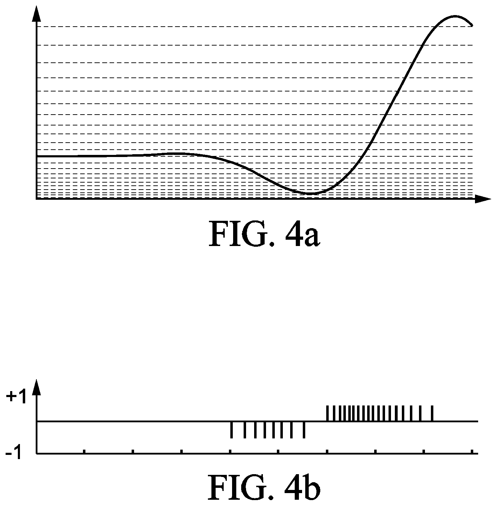

Le seuil d'activation Q peut être fixe, comme dans le cas des figures 3a - 3c, ou adaptatif en fonction de l'intensité lumineuse, comme dans le cas des figures 4a et 4b. Par exemple, le seuil ±Q peut être comparé aux variations du logarithme de l'intensité lumineuse pour la génération d'un événement ±1.

La classe de capteurs photosensibles asynchrones générant des évènements à partir des variations d'intensité lumineuse est désignée par l'acronyme DVS, pour « Dynamique Vision Sensor » (capteur de vision dynamique).

À titre d'exemple, le capteur de vision asynchrone (13) peut être un capteur DVS du type décrit dans "A 128x128 120 dB 15 µs Latency Asynchronous Temporal Contrast Vision Sensor", P. Lichtsteiner, et al., IEEE Journal of Solid-State Circuits, Vol. 43, No. 2, février 2008, pp. 566-576, ou dans la demande de brevet US 2008/0135731 A1.

Une autre génération de capteurs photosensibles asynchrones permet de générer de l'information asynchrone indiquant des événements ainsi qu'une caractéristique associée, par exemple un niveau de gris.

L'article de Posch, C., Matolin, D., et Wohlgenannt, R. (2011) intitulé "A qvga 143 db dynamic range frame-free pwm image sensor with lossless pixel-level video compression and time-domain cds", et publié dans le IEEE Journal of Solid-State Circuits, 46, pages 259 -275. doi:10.1109/JSSC.2010.2085952, fournit une description d'exemples d'évènements codés par des niveaux de gris.

L'information asynchrone pour chaque pixel consiste là-encore en une succession d'impulsions ou raies positionnées dans le temps aux instants *tₖ* dépendant du profil lumineux pour le pixel. Chaque évènement peut par exemple correspondre à deux impulsions successives, la première indiquant l'instant de l'évènement et la seconde permettant de déterminer un niveau de gris pour le pixel en fonction de l'écart temporel entre les deux impulsions. L'information correspondant à un évènement pour un pixel comprend ainsi une première information relative à un instant d'occurrence de l'événement, et une deuxième information relative à une caractéristique lumineuse (niveau de gris) pour le pixel à cet instant.

Par exemple, la détection d'un évènement pour un pixel donné pourra se traduire par la génération par le capteur (13) d'une première impulsion signée identifiant l'évènement (par exemple évènement positif pour une illumination du pixel croissante, et évènement négatif pour une illumination du pixel décroissante), et une deuxième impulsion pour caractériser la quantité de lumière détectée : si la variation de lumière est lente, l'écart entre les deux impulsions sera long, ce qui traduira une faible quantité de lumière, et donc un niveau de gris sombre. Il y aura eu suffisamment de variation de lumière pour déclencher la détection d'un événement, mais peu de lumière au cours de cette variation. A l'inverse, si la variation de lumière est rapide, l'écart entre les deux impulsions sera faible, ce qui traduira une forte quantité de lumière, et donc un niveau de gris faible.

Le train d'impulsions généré par le capteur (13) pourra être transmis à l'unité de traitement (14), qui générera sur cette base des informations d'évènements pour le pixel, comme par exemple des informations d'écart temporel avec l'évènement précédent pour le pixel, de niveau de gris associé à l'évènement pour le pixel, de position (par exemple sous la forme de coordonnées (x,y) dans la matrice de pixels) du pixel pour lequel l'évènement a été détecté, et de sens de la variation de lumière détectée pour le pixel (croissante ou décroissante).

Ces informations d'évènements pour chaque pixel seront multiplexées pour générer un signal de sortie (15) de la caméra asynchrone (10).

A titre d'exemple, la caméra asynchrone (10) pourra incorporer dans un mode de réalisation un capteur de vision asynchrone basé sur évènements (13) de nouvelle génération, que l'on désigne parfois sous l'acronyme ATIS, pour « Asynchronous, Time-Based Image Sensor ». La caméra asynchrone et le capteur ATIS qu'elle incorpore pourront par exemple être du type de celle décrite dans l'article de C. Posch et al., intitulé « An Asynchronous Time-based Image Sensor » (IEEE International Symposium on Circuits and Systems, 2008, pages 2130-2133), ou bien du type de celle décrite dans l'article de C. Posch et al., intitulé « A QVGA 143 dB dynamic range frame-free PWM image sensor with lossless pixel-level video compression and time-domain CDS » (46(1) :259275, 2011).

Un autre exemple de capteur de vision ATIS utilisable dans le système d'imagerie proposé est décrit dans l'article de T. Delbruck, P. Lichsteiner, et C. Posch, intitulé « A 128x128 120 dB 15µs latency asynchronous temporal contrast vision sensor » (IEEE Journal of Solid-State Circuits, Vol. 43, NO. 2, Feb. 2008, p. 566 - 576). Ce capteur CMOS, qui dispose d'une matrice de 128 x 128 pixels, permet d'atteindre une dynamique supérieure à 120 dB pour une résolution temporelle de 15 µs.

Pour une caméra asynchrone, la résolution temporelle pourra donc être définie par l'écart temporel minimum entre deux impulsions que pourra générer la caméra en fonction des variations d'intensité lumineuse pour un pixel. Les exemples mentionnés ci-dessus indiquent que pour une caméra ATIS, la résolution temporelle atteinte peut être de l'ordre de la µs, soit un gain significatif par rapport aux caméras classiques (fonctionnant avec une horloge trame ou une horloge pixel).

Ce gain significatif de résolution temporelle est mis à profit pour la mise en oeuvre des systèmes d'imagerie proposés qui peuvent offrir des performances inégalées pour l'observation et l'analyse de phénomènes dynamiques. C'est le cas par exemple dans le domaine de l'observation et de l'analyse de la microcirculation sanguine, où l'emploi de caméras asynchrones dans un système d'imagerie peut permettre le mesure de paramètres physiologiques de cellules sanguines (et, en particulier, de globules rouges - en anglais, RBC pour Red Blood Cells) tels que la vitesse, la trajectoire, et la quantité de lumière renvoyée à différentes longueurs d'onde.

De même, la performance en dynamique que l'on peut obtenir avec un capteur du type DVS ou ATIS est largement supérieure à celle que l'on peut atteindre avec une caméra vidéo classique ayant une fréquence d'échantillonnage réaliste. Par exemple, un capteur de ce type permet d'atteindre des résolutions temporelles de l'ordre de la microseconde avec une gamme de luminance supérieure à 120 dB, ce qui est très supérieur à une caméra standard CMOS/CCD qui possède typiquement une gamme de luminance de 60-70 dB.

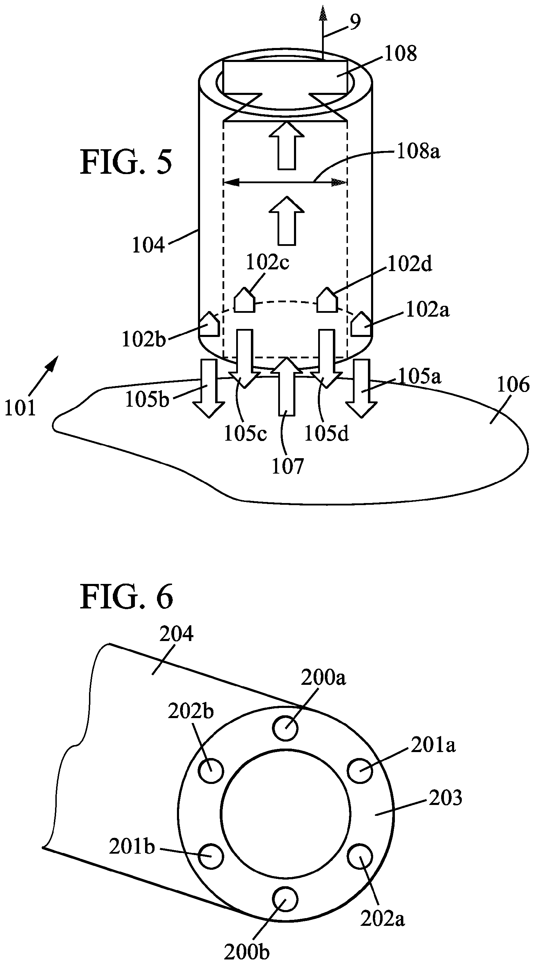

La figure 5 montre un schéma de système d'imagerie utilisant le principe de la microscopie en champ sombre selon un mode de réalisation. Le système d'imagerie (100) illustré comprend un support optique (104) pouvant être agencé en un cylindre creux. Sur l'une des extrémités du cylindre du support optique (104) sont montées une ou plusieurs diodes électroluminescentes (102a - 102d) agencées sur la section du cylindre de manière à former un anneau d'illumination du corps (106) à analyser en projetant une pluralité de signaux optiques d'intensités lumineuses respectives sensiblement constantes.

Ces diodes LEDs (102a - 102d) formant un ensemble de sources lumineuses pourraient être remplacées ou complétées par des diodes laser dans un autre mode de réalisation. Les sources à LED présentent l'avantage de permettre la génération d'un signal optique à spectre large, propriété utile par exemple pour effectuer une analyse de vitesse des cellules sanguines dans une application d'analyse de microcirculation. Le signal émis par une source laser sera plus resserré spectralement.

Ces sources lumineuses sont ainsi agencées sur le support optique (104) pour projeter chacune un signal optique d'excitation (105a - 105d) en direction du corps (106) à analyser lors de l'utilisation du système (101).

Dans un mode de réalisation, des traitements optiques peuvent être effectués sur les signaux émis par les sources lumineuses (par exemple focalisation, amplification etc.) de sorte que le signal d'excitation projeté sur le corps à observer est indirectement issu des sources lumineuses.

Le support optique (104) est en outre agencé pour recevoir la lumière rétrodiffusée (107) par le corps (106) à analyser à l'intérieur du cylindre creux, ce tube intérieur formant une zone sombre dans laquelle la lumière rétrodiffusée (107) est acheminée vers la caméra asynchrone (108). La caméra asynchrone (108) est positionnée à l'extrémité opposée du cylindre creux formant support optique (104), de celle où sont agencées les sources lumineuses (102a - 102d), de manière à ce que la lumière rétrodiffusée viennent traverser son optique d'acquisition (108a) et illuminer son capteur de vision lors de l'utilisation du système (101). La caméra (108) est en outre munie d'une interface (109) de sortie d'un signal d'imagerie sur lequel différents traitements peuvent être effectués pour l'application envisagée.

Cette configuration du support optique (104) permet de créer un anneau lumineux qui vient éclairer les tissus à analyser dans le cas d'une application d'analyse de microcirculation en champ sombre, ainsi qu'une zone sombre dans laquelle la lumière rétrodiffusée est acheminée, permettant ainsi un meilleur contraste lorsque le système est positionné à proximité du corps à analyser, en minimisant l'éblouissement lié à la rétrodiffusion des couches supérieures du corps étudié.

En effet, le dispositif permet de projeter une grande quantité de lumière par le biais de l'anneau lumineux tout en n'analysant que les parties du corps étudié qui ne sont pas directement illuminées, ce par le biais de l'analyse de la lumière rétrodiffusée issue de la zone centrale de l'anneau d'illumination.

Dans un mode de réalisation, les sources de lumière (102a - 102d) sont agencées pour émettre respectivement de la lumière rouge, verte, et/ou infrarouge, en fonction des post-traitements effectués. On pourra par exemple, configurer les sources de lumières pour qu'une première source émette de la lumière rouge, une deuxième source émette de la lumière verte, et une troisième source émette de la lumière infrarouge. Les sources de lumière pourront donc être configurées pour émettre, ensemble lors de l'utilisation du dispositif, des signaux lumineux à différentes longueurs d'onde respectives. En effet, on observe que la quantité de lumière absorbée par les tissus dans le cas de l'analyse de la microcirculation dépend de la longueur d'onde émise. Par exemple, on pourra utiliser un éclairage en lumière rouge et en lumière infra-rouge pour analyser l'oxygénation du sang.

Bien que la figure 5 illustre un schéma de dispositif d'imagerie fonctionnant selon le principe de la microscopie en champ sombre, le dispositif proposé n'est pas limité à une technologie d'imagerie spécifique, et pourrait mettre en oeuvre, dans d'autres modes de réalisation, tous types de technologies d'imagerie, comme par exemple la technologie d'imagerie à analyse de polarisation (OPS).

Dans un mode de réalisation illustré sur la figure 6, le dispositif d'imagerie proposé comprendra au moins 6 sources lumineuses, dont 2 sources de lumière rouge (200a, 200b), 2 sources de lumière verte (201a, 201b), et 2 sources de lumière infra-rouge (202a, 202b), par exemple montée sur l'extrémité (203) d'un cylindre creux d'un support optique (204) du dispositif.

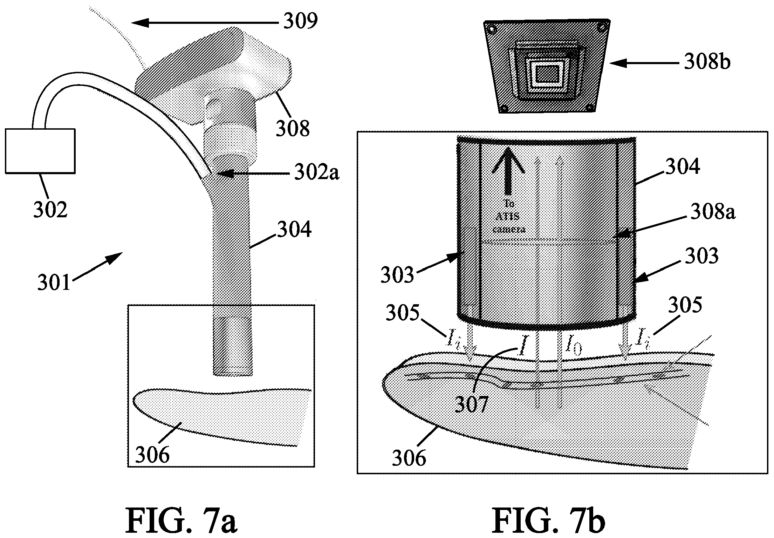

La figure 7a montre une variante du dispositif de la figure 5, dans laquelle une ou plusieurs sources de lumière (302), sont couplées optiquement au support optique (304) du dispositif par l'intermédiaire de fibres optiques (302a). Le support optique est conformé en un cylindre creux, sur l'une des extrémités duquel est montée par couplage mécanique une caméra asynchrone (308). Le signal généré par la caméra asynchrone (308) est produit sur une interface de sortie (309) de la caméra (308), par exemple de type connexion USB.

La figure 7b est une vue schématique partielle du dispositif (301) de la figure 7a, et montre un capteur de vision ATIS (308b) et l'optique d'acquisition (308a) de la caméra ATIS (308) placée au foyer principal de cette optique d'acquisition (308a), de manière par exemple à imager une cellule sanguine d'un corps observé (306) sur un bloc de 3 x 3 pixels.

La figure 7b montre en outre la structure interne du support optique (304) dans laquelle sont agencés un ou plusieurs guides de lumière couplés à des connexions d'entrée de fibre optique ou, selon le mode de réalisation, un ou plusieurs conduits conformés pour y introduire une extrémité de fibre optique (303), pour amener la lumière incidente issue de la ou des sources de lumières (302). Le support optique (304) est ainsi couplé optiquement à la source de lumière (302) et agencé pour projeter un signal d'excitation lumineux d'intensité lumineuse sensiblement constante (305) (lumière incidente lᵢ) issu de la source de lumière (302) vers un corps (306) à observer lors de l'utilisation du dispositif (300). La lumière rétrodiffusée l et l₀ (307) est acheminée par la partie intérieure du cylindre du support optique (304) vers le capteur ATIS (308b).

Bien que les figures 7a et 7b illustrent un dispositif d'imagerie fonctionnant selon le principe de la microscopie en champ sombre, le dispositif proposé n'est pas limité à une technologie d'imagerie spécifique, et pourrait mettre en oeuvre dans d'autres modes de réalisation tous types de technologie d'imagerie, comme par exemple la technologie d'imagerie à analyse de polarisation (OPS).

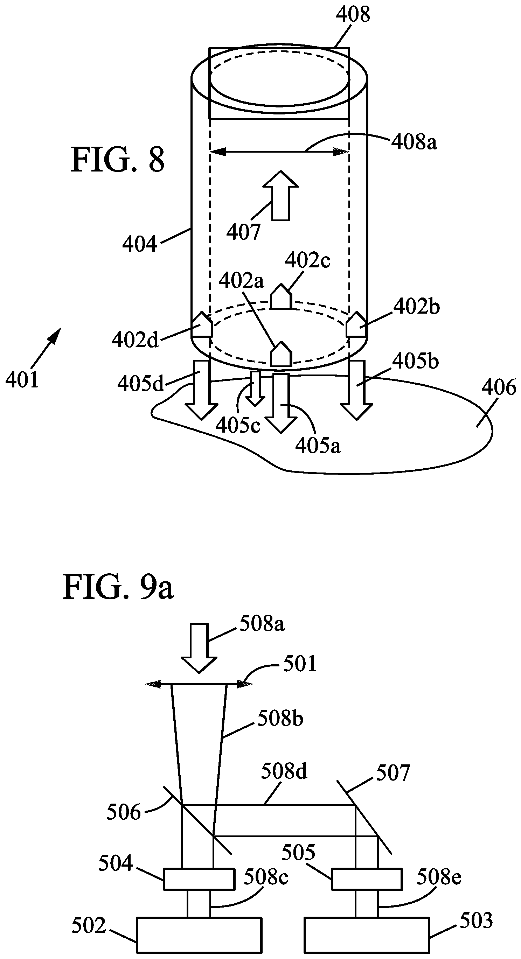

La figure 8 montre un mode de réalisation dans lequel le dispositif d'imagerie comprend une pluralité de caméras ATIS. Les caméras ATIS du dispositif sont agencées spatialement de manière à capter sensiblement le même signal de lumière rétrodiffusée par le corps à observer pour le pixel.

La figure 8 est un schéma illustrant le dispositif d'imagerie (401) fonctionnant selon le principe de la microscopie en champ sombre. Le système d'imagerie (401) illustré comprend un support optique (404) agencé en un cylindre creux. Sur l'une des extrémités du cylindre du support optique (404) sont montées une ou plusieurs sources de lumière (402a - 402d), par exemple des diodes électroluminescentes et/ou des diodes laser, agencées sur la section du cylindre de manière à former un anneau d'illumination du corps (406) à analyser, en projetant une pluralité de signaux d'excitation lumineuse d'intensité sensiblement constante. Ces sources lumineuses (402a - 402d) sont ainsi agencées sur le support optique (404) pour projeter chacune un signal optique d'excitation (405a - 405d) en direction du corps (406) à analyser lors de l'utilisation du système (401). Dans un mode de réalisation, des traitements optiques peuvent être effectués sur les signaux émis par les sources lumineuses (par exemple focalisation, amplification etc.) de sorte que les signaux d'excitation projetés sur le corps à observer sont respectivement indirectement issus des sources lumineuses (402a - 402d).

Le support optique (404) est en outre agencé pour recevoir la lumière rétrodiffusée (407) par le corps (406) à analyser à l'intérieur du cylindre creux, ce tube intérieur formant une zone sombre dans laquelle la lumière rétrodiffusée (407) est acheminée vers le bloc de capteurs asynchrones (408) lors de l'utilisation du dispositif. Dans le mode de réalisation illustré, le bloc de capteurs asynchrones (408) est positionné à l'extrémité opposée du cylindre creux formant support optique (404) de celle où sont agencées les sources lumineuses (402a - 402d), de manière à ce que la lumière rétrodiffusée (407) vienne traverser une optique d'acquisition (408a) et illuminer les capteurs de vision du bloc de capteurs asynchrones (408) lors de l'utilisation du système (401). Les capteurs asynchrones du bloc (408) peuvent donc utiliser une même optique d'acquisition (408a) dans un mode de réalisation. Ce mode de réalisation n'est toutefois pas limitatif, et chaque caméra asynchrone du dispositif peut avoir sa propre optique d'acquisition tout en étant agencée pour capter des évènements asynchrones correspondant à des variations de la lumière rétrodiffusée (407) par le corps à observer (406) pour chaque pixel de son capteur de vision.

Les caméras asynchrones sont en outre chacune munie d'une interface de sortie (non représentée sur la figure) d'un signal d'imagerie, sur lequel différents traitements peuvent être effectués pour l'application envisagée.

Bien que la figure 8 illustre un schéma de dispositif d'imagerie multi-caméras fonctionnant selon le principe de la microscopie en champ sombre, le dispositif proposé n'est pas limité à une technologie d'imagerie spécifique, et pourrait mettre en oeuvre, dans d'autres modes de réalisation, tous types de technologies d'imagerie, comme par exemple la technologie OPS.

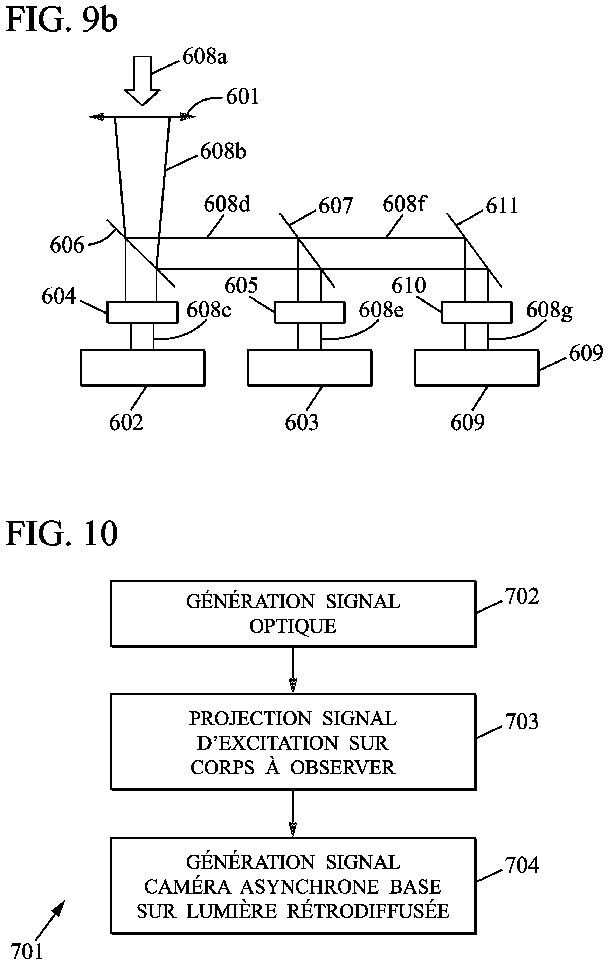

Les figures 9a et 9b illustrent un exemple de réalisation de blocs de deux et trois capteurs asynchrones, respectivement.

En référence à la figure 9a, la lumière rétrodiffusée (508a) par le corps à observer traverse une optique d'acquisition (501) de caméra asynchrone du dispositif d'imagerie proposé et produit un faisceau lumineux (508b), qui vient en partie traverser et en partie se réfléchir sur un miroir dichroïque (506) agencé pour scinder le faisceau lumineux (508b) issu de l'optique d'acquisition (501) en deux faisceaux. La partie du faisceau lumineux (508b) non réfléchie par le miroir dichroïque (506) est traitée par un bloc de traitement optique (504), par exemple pour effectuer un filtrage optique du faisceau lumineux (508b), qui génère en sortie un faisceau lumineux (508c) qui vient illuminer un premier capteur de vision asynchrone (502). La partie (508d) du faisceau lumineux (508b) réfléchie par le miroir dichroïque (506) vient se réfléchir sur un miroir (507) agencé pour rediriger le faisceau lumineux (508d) issu de l'optique d'acquisition (501) et du miroir dichroïque (506) vers un deuxième ensemble bloc de traitement optique (505) et capteur de vision asynchrone (503). Le faisceau réfléchi (508d) est ainsi traité par un bloc de traitement optique (505), par exemple pour effectuer un filtrage optique du faisceau lumineux (508d), qui génère en sortie un faisceau lumineux (508e) qui vient illuminer un deuxième capteur de vision asynchrone (503).

Ces ensembles bloc de traitement optique et capteur de vision asynchrone permettent de sélectionner certaines caractéristiques du signal qui vient illuminer chaque capteur de vision asynchrone, tout en assurant que chaque signal provient de la lumière rétrodiffusée par le corps à observer, de sorte que chaque capteur observe sensiblement la même scène. Ainsi, chaque évènement détecté par les caméras asynchrones correspond à un même corps. Par exemple, dans le cadre d'une application d'observation et d'analyse de la microcirculation, chaque évènement détecté relativement à une même cellule sanguine à un instant donné le sera par chacun des capteurs asynchrones du dispositif d'imagerie. Le dispositif d'imagerie proposé dans le mode de réalisation multi-caméras asynchrones est ainsi agencé pour capturer des informations sur le même pixel (en supposant une correspondance entre les pixels des matrices des différents capteurs de vision asynchrones) et à un même instant donné, ce qui permet de ne pas diminuer la résolution spatiale de l'observation (comme avec un filtre de Bayer par exemple) tout en conservant la résolution temporelle élevée obtenue avec des caméras asynchrones.

Par exemple, les blocs de traitement optique de la figure 9a peuvent comprendre un filtre optique pour ne laisser passer que la lumière rouge pour l'un, et que la lumière infra-rouge pour l'autre, afin d'acquérir des données d'imagerie permettant de déterminer un taux d'oxygénation de cellules sanguines dans une application d'analyse de la microcirculation.

Plus généralement, on pourra utiliser le mode de réalisation multi-caméras du dispositif d'imagerie proposé pour des analyses spectrales de la lumière rétrodiffusée par le corps à analyser, en utilisant pour chaque caméra des filtres optiques opérant sur une bande de fréquence à analyser.

En référence à la figure 9b, le bloc de capteurs de vision asynchrones peut comprendre trois ensembles traitement optique et capteur asynchrone. La lumière rétrodiffusée (608a) traverse une optique d'acquisition (601) qui génère un faisceau (608b) en partie réfléchi par un premier miroir dichroïque (606). La partie de faisceau (608b) non réfléchie par le premier miroir dichroïque (606) traverse un premier bloc de traitement optique (604) qui génère en sortie un faisceau (608c) qui vient illuminer un premier capteur de vision asynchrone (602).

La partie (608d) de faisceau (608b) réfléchie par le premier miroir dichroïque (606) est elle-même en partie réfléchie sur un deuxième miroir dichroïque (607) pour être redirigée vers un deuxième bloc de traitement optique (605) qui génère en sortie un faisceau (608e) qui vient illuminer un deuxième capteur de vision asynchrone (603).

La partie (608f) de faisceau (608d) non réfléchie par le deuxième miroir dichroïque (607) est réfléchie sur un troisième miroir (611) pour être redirigée vers un troisième bloc de traitement optique (610) qui génère en sortie un faisceau (608g) qui vient illuminer un troisième capteur de vision asynchrone (609).

Ce mode de réalisation du dispositif d'imagerie avec trois caméras asynchrones permet d'effectuer des post-traitements nécessitant une capture en parallèle d'imagerie de le corps à analyser.

En référence aux figures 9a et 9b, les miroirs dichroïques illustrés sont fournis à titre d'exemple de composant optique permettant de diviser un faisceau incident en deux faisceaux. D'autre part, l'agencement de tels composants optiques pourra varier en fonction de la configuration spatiale des capteurs de vision asynchrones ou des caméras asynchrones.

Dans un autre mode de réalisation, le dispositif d'imagerie proposé peut embarquer quatre caméras. Trois sources de lumière (rouge, vert et infra rouge) illuminent l'échantillon de manière indirecte avec une structure par exemple annulaire. La lumière rétrodiffusée par l'échantillon à analyser est alors captée par un système optique situé au-dessus d'une zone sombre pour un dispositif fonctionnant sur le principe de la microscopie en champ sombre. La lumière est ensuite répartie en fonction de sa longueur d'onde sur les 4 caméras, par exemple au moyen de filtres agencés sur le trajet de la lumière avant d'atteindre le capteur de chaque caméra : une caméra asynchrone pour le rouge, une caméra asynchrone pour l'infra-rouge, une caméra asynchrone pour le vert, et une caméra classique à haute résolution spatiale (par exemple 4 M pixels pour une résolution temporelle de 300 trames par sec.) pour le vert également.

La figure 10 illustre le procédé d'imagerie (701) proposé selon un mode de réalisation. Dans ce mode de réalisation, on génère (702) un signal optique duquel on extrait un signal d'excitation lumineux d'intensité lumineuse sensiblement constante. Le signal d'excitation lumineux peut être directement issu du signal optique, ou l'être indirectement, après certains traitements (focalisation, traitement fréquentiel, traitement de polarisation, etc.). Le signal d'excitation lumineux est projeté (703) sur le corps à observer, afin de capter la lumière rétrodiffusée par ce corps. L'utilisation d'au moins un capteur asynchrone permet de générer (704) un signal comprenant, pour chaque pixel de la matrice de pixels du capteur, une séquence de signal représentant des évènements asynchrones correspondant à des variations de la lumière rétrodiffusée par le corps à observer pour le pixel.

Bien que décrits à travers un certain nombre d'exemples de réalisation détaillés, le procédé de commande d'activation et l'équipement pour la mise en oeuvre du procédé comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

De plus, différents aspects et caractéristiques décrits ci-dessus peuvent être mis en oeuvre ensemble, ou séparément, ou bien substitués les uns aux autres, et l'ensemble des différentes combinaisons et sous combinaisons des aspects et caractéristiques font partie de la portée de l'invention. En outre, il se peut que certains systèmes et équipements décrits ci-dessus n'incorporent pas la totalité des modules et fonctions décrits pour les modes de réalisation préférés.

Les informations et signaux décrits dans le présent document peuvent être représentés selon une multitude de technologies et de techniques. Par exemple, les instructions, messages, données, commandes, informations, signaux, bits et symboles peuvent être représentés par des tensions, intensités, ondes électromagnétiques ou une combinaison de ces derniers.

En fonction du mode de réalisation choisi, certains actes, actions, évènements ou fonctions de chacune des méthodes décrites dans le présent document peuvent être effectués ou se produire selon un ordre différent de celui dans lequel ils ont été décrits, ou peuvent être ajoutés, fusionnés ou bien ne pas être effectués ou ne pas se produire, selon le cas. En outre, dans certains modes de réalisation, certains actes, actions ou évènements sont effectués ou se produisent concurremment et non pas successivement.

## Revendications

1. Dispositif d'imagerie (1, 101, 401) comprenant :
∘ au moins une source de lumière (2, 102a - 102d, 402a - 402d) agencée pour générer un signal optique (3),
**caractérisé en ce qu'**il contient en outre :
∘ un support optique (4, 104, 404) couplé à la source de lumière (2, 102a - 102d, 402a - 402d) et agencé pour projeter un signal d'excitation lumineux (5, 105a - 105d, 405a - 405d) d'intensité lumineuse sensiblement constante issu de la source de lumière (2, 102a - 102d, 402a - 402d) vers un corps (6, 106, 406) à observer lors de l'utilisation du dispositif (1, 101, 401); et
∘ une première caméra asynchrone (8, 108, 602) couplée au support optique (4, 104, 404) et configurée pour générer un signal comprenant, pour chaque pixel d'une première matrice de pixels, une séquence de signal représentant des évènements asynchrones correspondant à des variations de la lumière rétrodiffusée (7, 107, 407, 608b) par le corps (6, 106, 406) à observer pour le pixel.

2. Dispositif (1, 101, 401) selon la revendication 1, comprenant une deuxième caméra asynchrone (603) couplée au support optique (4, 104, 404) et configurée pour générer un signal comprenant, pour chaque pixel d'une deuxième matrice de pixels, une séquence de signal représentant des évènements asynchrones correspondant à des variations de la lumière rétrodiffusée (7, 107, 407, 608b) par le corps (6, 106, 406) à observer pour le pixel, et dans lequel un premier filtre optique est agencé pour filtrer la lumière rétrodiffusée (7, 107, 407, 608b) captée par la première caméra asynchrone (8, 108, 602).

3. Dispositif (1, 101, 401) selon la revendication 2, dans lequel un deuxième filtre optique est agencé pour filtrer la lumière rétrodiffusée (7, 107, 407, 608b) captée par la deuxième caméra asynchrone (603).

4. Dispositif (1, 101, 401) selon la revendication 3, comprenant une troisième caméra asynchrone (609) couplée au support optique (4, 104, 404) et configurée pour générer un signal comprenant, pour chaque pixel d'une troisième matrice de pixels, une séquence de signal représentant des évènements asynchrones correspondant à des variations de la lumière rétrodiffusée (7, 107, 407, 608b) par le corps (6, 106, 406) à observer pour le pixel, et dans lequel un troisième filtre optique est agencé pour filtrer la lumière rétrodiffusée (7, 107, 407, 608b) captée par la troisième caméra asynchrone (609).

5. Dispositif (1, 101, 401) selon l'une quelconque des revendications précédentes, dans lequel la source de lumière (2, 102a - 102d, 402a - 402d) est une diode électroluminescente ou une source laser.

6. Dispositif (1, 101, 401) selon l'une quelconque des revendications précédentes, comprenant une pluralité de sources de lumière (2, 102a - 102d, 402a - 402d) agencées sur le support optique (4, 104, 404) pour générer des signaux d'excitation lumineux d'intensités lumineuses respectives sensiblement constantes.

7. Dispositif (1, 101, 401) selon la revendication 6, dans lequel les sources de lumière (2, 102a - 102d, 402a - 402d) sont en outre agencées sur le support optique (4, 104, 404) de manière à former un anneau lumineux.

8. Dispositif (1, 101, 401) selon l'une quelconque des revendications précédentes, dans lequel la caméra asynchrone (8, 108, 602) est agencée sur le support optique (4, 104, 404) située au-dessus d'une zone non illuminée par la source de lumière (2, 102a - 102d, 402a - 402d).

9. Système d'imagerie de la microcirculation comprenant un dispositif (1, 101, 401) selon l'une quelconque des revendications 1 à 8.

10. Procédé d'imagerie (701), comprenant :
∘ générer (702) un signal optique (3);
**caractérisé en ce qu'**il comprend en outre :
∘ projeter (703) un signal d'excitation lumineux (5, 105a - 105d, 405a - 405d) d'intensité lumineuse sensiblement constante issu du signal optique (3) vers un corps (6, 106, 406) à observer ; et
∘ générer (704) un signal comprenant, pour chaque pixel d'une première matrice de pixels, une séquence de signal représentant des évènements asynchrones correspondant à des variations de la lumière rétrodiffusée (7, 107, 407, 608b) par le corps (6, 106, 406) à observer pour le pixel.

## Patentansprüche

1. Bildgebungsvorrichtung (1, 101, 401) aufweisend:
∘ mindestens eine Lichtquelle (2, 102-102d, 402a-402d) zum Erzeugen eines optischen Signals (3),
**dadurch gekennzeichnet, dass** sie ferner aufweist:
∘ einen optischen Träger (4, 104, 404), der mit der Lichtquelle (2, 102-102d, 402a-402d) gekoppelt ist und eingerichtet ist, ein aus der Lichtquelle (2, 102-102d, 402a-402d) stammendes Anregungslichtsignal (5, 105a-105d, 405a-405d), das eine im Wesentlichen konstante Lichtintensität hat, hin zu einem Körper (6, 106, 406), der unter Verwendung der Vorrichtung (1, 101, 401) beobachtet werden soll, zu projizieren; und
∘ eine erste Asynchronkamera (8, 108, 602), die mit dem optischen Träger (4, 104, 404) gekoppelt ist und konfiguriert ist, ein Signal zu erzeugen, das für jedes Pixel einer ersten Pixelmatrix eine Signalsequenz aufweist, die asynchrone Ereignisse repräsentiert, die Variationen des von dem zu beobachtenden Körper (6, 106, 406) zurückgestreuten Lichts (7, 107, 407, 608b) für das Pixel entsprechen.

2. Vorrichtung (1, 101, 401) nach Anspruch 1, aufweisend eine zweite Asynchronkamera (603), die mit dem optischen Träger (4, 104, 404) gekoppelt ist und konfiguriert ist, ein Signal zu erzeugen, das für jedes Pixel einer zweiten Pixelmatrix eine Signalsequenz aufweist, die asynchrone Ereignisse repräsentiert, die Variationen des von dem zu beobachtenden Körper (6, 106, 406) zurückgestreuten Lichts (7, 107, 407, 608b) für das Pixel entsprechen, und in welcher ein erstes optisches Filter angeordnet ist, um das von der ersten Asynchronkamera (8, 108, 602) eingefangene zurückgestreute Licht (7, 107, 407, 608b) zu filtern.

3. Vorrichtung (1, 101, 401) nach Anspruch 2, wobei ein zweites optisches Filter angeordnet ist, um das von der zweiten Asynchronkamera (603) eingefangene zurückgestreute Licht (7, 107, 407, 608b) zu filtern.

4. Vorrichtung (1, 101, 401) nach Anspruch 3, aufweisend eine dritte Asynchronkamera (609), die mit dem optischen Träger (4, 104, 404) gekoppelt ist und konfiguriert ist, ein Signal zu erzeugen, das für jedes Pixel einer dritten Pixelmatrix eine Signalsequenz aufweist, die asynchrone Ereignisse repräsentiert, die Variationen des von dem zu beobachtenden Körper (6, 106, 406) zurückgestreuten Lichts (7, 107, 407, 608b) für das Pixel entsprechen, und in welcher ein drittes optisches Filter angeordnet ist, um das von der dritten Asynchronkamera (609) eingefangene zurückgestreute Licht (7, 107, 407, 608b) zu filtern.

5. Vorrichtung (1, 101, 401) nach einem der vorstehenden Ansprüche, wobei die Lichtquelle (2, 102a-102d, 402a-402d) eine Elektrolumineszenz-Diode oder eine Laserquelle ist.

6. Vorrichtung (1, 101, 401) nach einem der vorstehenden Ansprüche, aufweisend mehrere an dem optischen Träger (4, 104, 404) angeordnete Lichtquellen (2, 102a-102d, 402a-402d) zum Erzeugen von Anregungslichtsignalen, die jeweils im Wesentlichen konstante Lichtintensitäten haben.

7. Vorrichtung (1, 101, 401) nach Anspruch 6, wobei die Lichtquellen (2, 102a-102d, 402a-402d) an dem optischen Träger (4, 104, 404) ferner so angeordnet sind, um einen Lichtring zu bilden.

8. Vorrichtung (1, 101, 401) nach einem der vorstehenden Ansprüche, wobei die Asynchronkamera (8, 108, 602) an dem optischen Träger (4, 104, 404) oberhalb einer von der Lichtquelle (2, 102a-102d, 402a-402d) nicht beleuchteten Zone angeordnet ist.

9. Bildgebungssystem zum Abbilden der Mikro-Blutzirkulation, aufweisend eine Vorrichtung (1, 101, 401) nach einem der Ansprüche 1 bis 8.

10. Bildgebungsverfahren (701), aufweisend:
o Erzeugen (702) eines optischen Signals (3);
**dadurch gekennzeichnet, dass** es ferner aufweist:
∘ Projizieren (703) eines aus dem optischen Signal (3) stammenden Anregungslichtsignals (5, 105a-105d, 405a-405d), das eine im Wesentlichen konstante Lichtintensität hat, hin zu einem zu beobachtenden Körper (6, 106, 406); und
Erzeugen (704) eines Signals, das für jedes Pixel einer ersten Pixelmatrix eine Signalsequenz aufweist, die asynchrone Ereignisse repräsentiert, die Variationen des von dem zu beobachtenden Körper (6, 106, 406) zurückgestreuten Lichts (7, 107, 407, 608b) für das Pixel entsprechen.

## Claims

1. Imaging device (1, 101, 401) comprising:
- at least one light source (2, 102a - 102d, 402a - 402d) arranged so as to generate an optical signal;
**characterized in that** it further comprises:
- an optical support (4, 104, 404) coupled to the light source (2, 102a - 102d, 402a - 402d) and arranged so as to project a luminous excitation signal (5, 105a - 105d, 405a - 405d) with a substantially constant light intensity from the light source (2, 102a - 102d, 402a - 402d) to a body (6, 106, 406) to be observed when using the device (1, 101, 401); and
- a first asynchronous camera (8, 108, 602) coupled to the optical support (4, 104, 404) and configured so as to generate a signal comprising, for each pixel of a first pixel array, a signal sequence representing asynchronous events corresponding to variations in the light (7, 107, 407, 608b) backscattered by the body (6, 106, 406) to be observed for the pixel.

2. Device (1, 101, 401) according to claim 1, comprising a second asynchronous camera (603) coupled to the optical support (4, 104, 404) and configured so as to generate a signal comprising, for each pixel of a second pixel array, a signal sequence representing asynchronous events corresponding to variations in the light (7, 107, 407, 608b) backscattered by the body (6, 106, 406) to be observed for the pixel, and wherein a first optical filter is arranged so as to filter the backscattered light captured by the first asynchronous camera (8, 108, 602).

3. Device (1, 101, 401) according to claim 2, wherein a second optical filter is arranged so as to filter the backscattered light (7, 107, 407, 608b) captured by the second asynchronous camera (603).

4. Device (1, 101, 401) according to claim 3, comprising a third asynchronous camera (609) coupled to the optical support (4, 104, 404) and configured so as to generate a signal comprising, for each pixel of a third pixel array, a signal sequence representing asynchronous events corresponding to variations in the light (7, 107, 407, 608b) backscattered by the body (6, 106, 406) to be observed for the pixel, and wherein a third optical filter is arranged so as to filter the backscattered light (7, 107, 407, 608b) captured by the third asynchronous camera.

5. Device (1, 101, 401) according to any of the previous claims, wherein the light source (2, 102a - 102d, 402a - 402d) is a light-emitting diode or a laser source.

6. Device (1, 101, 401) according to any of the previous claims, comprising a plurality of light sources (2, 102a - 102d, 402a - 402d) arranged on the optical support (4, 104, 404) so as to generate luminous excitation signals with substantially constant respective light intensities.

7. Device (1, 101, 401) according to claim 6, wherein the light sources (2, 102a - 102d, 402a - 402d) are also arranged on the optical support (4, 104, 404) such that they form a luminous ring.

8. Device (1, 101, 401) according to any of the previous claims, wherein the asynchronous camera (8, 108, 602) is arranged on the optical support (4, 104, 404) located above an area not illuminated by the light source (2, 102a - 102d, 402a - 402d).

9. Microcirculation imaging system comprising a device (1, 101, 401) according to any one of claims 1 to 8.

10. Imaging method (701) comprising:
- generating (702) an optical signal (3);
**characterized in that** it further comprises:
- projecting (703) a luminous excitation signal (5, 105a - 105d, 405a - 405d) with a substantially constant light intensity from the optical signal (3) to a body (6, 106, 406) to be observed; and
- generating (704) a signal comprising, for each pixel of a first pixel array, a signal sequence representing asynchronous events corresponding to variations in the light (7, 107, 407, 608b) backscattered by the body (6, 106, 406) to be observed for the pixel.
